# EUROPEAN PATENT APPLICATION

(11) **EP 1 867 340 A1**
(43) Date of publication of application: **19.12.2007**
(21) Application number: 05720522.1
(22) Date of filing: 10.03.2005
(51) Int. Cl.: A61K 38/21, A61P 1/00, A61P 1/12

(54) **THERAPEUTIC AGENT FOR BOVINE DIGESTIVE SYSTEM DISEASE**

(71) Applicant: KABUSHIKI KAISHA HAYASHIBARA SEIBUTSU KAGAKU KENKYUJO, Okayama-shi, Okayama 700-0907 (JP); Biovet, Inc., Tokyo 150-0002 (JP)
(72) Inventor: TAMURA, Takatoshi, Tokyo 153-0044 (JP); TAKAHASHI, K., Nosuto-Meguro-Higashigaoka 205, Tokyo 152-0021 (JP); MIWA, Yoshikatsu, c/o Kabushiki Kaisha Hayashibara, Okayama-shi, Okayama 700-0907 (JP)
(74) Representative: Daniels, Jeffrey Nicholas
(86) International application number: PCT/JP2005/004251
(87) International publication number: WO 2006/095433

(57) **Abstract**

The objects of the present invention are to provide a therapeutic agent for bovine digestive system diseases and to provide a therapeutic method using the agent. The above objects are attained by providing a therapeutic agent for digestive system diseases, which comprises interferon as an effective ingredient and by a therapeutic method for bovine digestive system diseases using the therapeutic agent.

## Description

### TECHNICAL FIELD

The present invention relates to an animal drug for bovine, particularly, a therapeutic agent for bovine digestive system diseases comprising human interferon α (hereinafter, it may be abbreviated as "HuIFN-α") as an effective ingredient.

### BACKGROUND ART

Bovines are widely bred for beef or dairy cattle. Varieties of bovine have been created through longtime breed improvements. Among which, remarkably expensive varieties have been created. Nowadays some varieties are remarkably expensive. Bovines are so nervous as to tend to develop a digestive system disease that mainly excites diarrhea caused by transportation using autotrucks, change of feed or breeding environment, or infection with pathogenic microorganisms. Particularly, digestive system diseases caused by virus including rotavirus or coronavirus result in immense harm because of their high pathogenicity and infectiousness. Particularly for calf, the symptoms are so severe that the infected calf often glows slowly even after curing or even leads to death, and the economic loss is a large problem. At present, there is no reliable therapy for virus diseases of bovine. For the digestive system diseases, no therapy is actually provided other than symptomatic therapies such as avoiding dehydration, nutritional therapies for restoration of the strength, or administration of antibiotics for preventing of multiple or secondary infections (for example, US Patent No. 5910304). Development of the efficient therapies for bovine digestive system diseases, which also has small economic burden for the guardians, has been desired.

In these days, it is reported that oral administrations of a relatively small amount of interferon derived from the same or different species are curative for diseases of bovine, cat, swine, rat, mouse, and fowl (Japanese Patent Kokai No. 340549/1994 and Joseph M. Cummins and William E. Stewart II; Clinical Microbiology, vol. 18, No. 5, pp. 631-635 (1991)). The applicant of the present invention disclosed that therapeutic agent for cat respiratory diseases, comprising a small amount of HuIFN-α as an effective ingredient, and the therapy using the same in Japanese Patent Kokai No. 340549/1994, and disclosed that the method of combinational use of subtype α2 and subtype α8 of HuIFN-α has a beneficial effect in Japanese Patent Kokai No.201141/2002. However, the above documents do notmention that oral administrations of interferon can relive the diarrhea caused by the infection of bovine rotavirus or coronavirus, and HuIFN-α comprising certain subtypes is markedly effective for the digestive system diseases of bovine infected by these virus mainly causing diarrhea.

### DISCLOSURE OF INVENTION

An object of the present invention is to provide a therapeutic agent for bovine digestive system diseases that is efficient by administrations other than injection, such as oral or transnasal administration, leading to saving of the labor of persons in charge of the therapy or easing of the economic burden of guardians. Another object of the invention is to provide a means of a therapy for bovine digestive system diseases using the above agent.

The inventors of the present invention have dedicated to research on the therapy of bovine digestive system diseases using the human interferon (hereinafter, it may be abbreviated as "HuIFN") to attain the above objects. In consequence, it was found that a HuIFN-α drug, particularly comprising its subtype α8 in an amount of 10% or more by weight of the total amount of HuIFN-α (hereinafter, "%" means "% by weight" unless specified otherwise throughout the specification), has marked therapeutic effect on digestive system diseases of bovine including calf that mainly excite diarrhea. The present invention was accomplished by providing a therapeutic agent for bovine digestive system diseases and establishing a therapy using the agent.

### BEST MODE FOR CARRYING OUT THE INVENTION

HuIFN as referred to as in the present invention exhibits marked therapeutic effect on virus digestive system diseases of bovine even at a dose of 0.005 to 5,000 international units (hereinafter abbreviated as "IU") per kg of body weight of bovine.

HuIFN exerts high therapeutic effect on bovine digestive system diseases when administered orally or parenterally.

Bovine as referred to as in the present invention means animals that belong to the family Bovidae. When the agent of the present invention is used in a preventive manner, bovine means general animals belonging to the family *Bovidae,* which may develop digestive system diseases when infected with rotavirus, coronavirus, or virus that causes diarrhea. For therapeutic purpose, itmeans also animals that may develop symptoms mainly excite diarrhea by infection with the above virus or multiply or secondary infection with other virus, mycoplasma, bacteria, and parasitic worm, and it includes animals that have general clinical symptoms such as decreased appetite, hypodynamia, sparse fur, piloerection, or dehydration.

The therapeutic agent for bovine digestive system diseases of the present invention comprises HuIFN as an effective ingredient produced by human cells or by microorganisms or animal cells introduced with a HuIFN gene from a human cell. Nowadays, HuIFN is categorized mainly into three types, i.e., HuIFN-α, HuIFN-β, and HuIFN-γ based on the antigens, but any types of them can be used in the present invention although they have slightly different therapeutic effect on bovine digestive system diseases. Both HuIFN consisting of only one type and HuIFN comprising two or more types are usable for the above purpose. There are some subtypes in HuIFN-α, one or more subtypes are usable in combination. In each instance, HuIFN with high specific activity is desirable, and a purified product with a specific activity of 10⁵ IU/mg protein or more is preferable for oral administrations, and a purified product with a specific activity of 10⁷ IU/mg or more is preferable for parenteral administrations.

According to the test conducted by the inventors, among the above HuIFNs, HuIFN-α, particularly "naturally occurring type of HuIFN-α", that formed by human lymphoblastoid cell lines such as BALL-1 cells or Namalwa cells, has high therapeutic effects on bovine digestive system diseases with lesser side effect. In particular, agents comprising subtype α8 of HuIFN-α (hereinafter, designated as "subtype α8*"*) in an amount of 10% or more of the total amount of HuIFN-α are preferable as regard to the effects, and agents comprising subtype α8 in an amount of 10 to 80% of the total amount of HuIFN-α and subtype α2 of HuIFN-α (hereinafter, designated as "subtype α2") in an amount of 20 to 90% of the total amount of HuIFN-α are more preferable, which have the high therapeutic effects, and agents comprising subtype α8 in an amount of 20 to 40% of the total amount of HuIFN-α and subtype α2 in an amount of 60 to 80% of the total amount of HuIFN-α are much more preferable, which have markedly high therapeutic effects. HuIFN-α from BALL-1 cells comprising subtype α2 in an amount of about 75% of the total amount of HuIFN-α and subtype α8 in an amount of about 25% of the total amount of HuIFN-α, which is in good balance, resulted in highest effects in the present invention. It was found that a combined use of HuIFN-α and HuIFN-β enhances the effects of HuIFN-α.

The therapeutic agent for bovine digestive system diseases of the present invention can be prepared in the form of a product consisting of HuIFN or a composition comprising HuIFN and other physiologically or pharmaceutically acceptable ingredients such as a carrier, vehicle, diluent, adjuvant, stabilizer, and if necessary, antifebrile, antiinflammatory drug, antibacterial drug, digestant, nutritional supplement, or feedstuff. The therapeutic agent of the present invention includes drugs in dose-unit form, which comprises HuIFN as an effective ingredient in an integral multiple amount (up to four times) or aliquot part (down to 1/4) of daily dosage and are physically dividable in adequate doses. When two or more types and/or subtypes of HuTFN-α are used in combination, the following forms are feasible; combinational use of agents comprising either types or subtypes of HuIFN-α alone, use of an agent comprising two or more types of HuIFN, or combinational use of both agents. Examples of the former of the above agents include a powdery drug, gelled oral drug, granular drug, liquid drug, tablet, capsule drug, sublingual drug, injectable drug or film-formed oral drug. The powdery or granular drugs can be used after dissolved in distilled water, saline, milk or imitation milk.

The following explains the dosage and usage of the therapeutic agent of the present invention for bovine digestive system diseases. The agent of the present invention gives desired preventive and/or therapeutic effects by oral or parenteral administrations. To be concrete, usually HuIFN can be administered at 0.005 to 5,000 IU/dose, preferably at 0.05 to 100 IU/dose, more preferably at 0.1 to 50 IU/dose per kg of body weight of bovine, one to three times a day or 1 to 5 times a week for one day to six months according to the symptoms and conditions of the patient, even though it depends on the kind of bovine, the kind of causal virus, symptom, dosage, usage, used type of HuIFN.

For oral or transnasal administrations of the agents of the present invention, the agents are prepared in oral-administrable form such as a powdery drug, troche, granular drug, liquid drug, tablet, sublingual drug, and feedstuff. They can be administered into the oral cavity, nasal cavity, esophagus, or stomach in conventional method, without modification or by dissolving or mixing in feed, milk, or imitation milk, or using apparatus for oral administration, spray, or stomach sonde. For parenteral administrations, the injectable agents of the present invention are injected intractaneously, subctaneously, intramuscularly, intravascularly (into the artery or vein), or intraperitoneally. According to the tests performed by the inventors, the therapeutic effects may not be obtained at a dosage below the dose described above, while at a dosage over the dose described above, antibody formation or side effects may become higher, or the economic burden of guardians may become higher compared to the effects, so the dosage described above is considered to be best.

If administration routes are compared in terms of therapeutic effect and easiness of administrations, oral administration has an advantage that it can be easily conducted at general breeding farm or repository of bovine. For a precise administration of prescribed amount of HuIFN, any of intravascular, intractaneous, subctaneous, or intramuscular administration is feasible.

The agent of the present invention exhibits preventive and therapeutic effects on bovine respiratory diseases, prevention of the body weight loss during transport, and acceleration of calf fattening, in addition to the therapeutic or preventive effects on bovine digestive system diseases. The agent is also effective for mammals or birds including horse, swine, cat, dog, mouse and fowl, as well as bovine. The agent of the present invention is usable not only as a therapeutic agent for bovine digestive system diseases but also as a preventive or therapeutic agent for various diseases, a preventive agent for body weight loss during transport, and a fattening-acceleration agent for the above general animals.

Throughout this specification, the activity of HuIFN was measured as follows: The restriction rates of HuIFN for cytopathic effect of sindbis virus to FL cells were measured by microtitor method, and the measured values were converted into the "IU" that is determined by using "Ga23-901-532" as a standard specimen, which is established as the international standard HuIFN by World Health organization.

### Experiment 1

### Preparation of standard HuIFN-α specimen and HuIFN-α agent

According to the method of Example 1 described below, a purified HuIFN-α (specific activity of 2×10⁸ IU/mg protein) was prepared from BALL-1 cells (hereinafter, this interferon may be abbreviated as "BALL-1 interferon"), and according to the method of Example 2, a purified recombinant subtype α8 (specific activity of 2×10⁸ IU/mg protein) and recombinant subtype α2 (specific activity of 7×10⁷ IU/mg protein) were prepared. Another HuIFN-α induced by allowing Sendai virus to act on leukocyte separated from human peripheral blood by conventional method (hereinafter, this interferon may be abbreviated as "leukocyte interferon") was purified by the method described in K, Cantell et. al.; The Journal of General Virology, Vol. 39, pp. 541-543 (1978) to give a partially purified leukocyte interferon having a specific activity of 2×10⁶ IU/mg protein. The partially purified HuIFN was further purified by antibody-chromatography using a HuIFN-α monoclonal antibody, then applied to gel-chromatography equilibrated with phosphate buffer (pH7.0) containing human albumin at a concentration of about 0.1 mg/ml to give a purified leukocyte interferon (specific activity of 2×10⁸ IU/mg protein). According to the method of Example 5 described below, these two kinds of HuIFN, recombinant subtypes α8 and α2 were made into a powdery agent containing each HuIFN-α at a dose of 200 IU/g by using "FINETOSE^{®}" (anhydrous crystalline maltose commercialized by Hayashibara Shoji, Inc.) as a filler. For subtypes, seven kinds of powdery agent, which contain subtypes α8 and α2 at a mass ratio of 10:0, 9:1, 8:2, 4:6, 2:8, 1:9, or 0:10 were prepared.

### Experiment 2

### Clinical test

A hundred of bovines infected with rotavirus and developing diarrhea were orally administrated with the HuIFN-α agents prepared in Experiment 1 once a day, and the therapeutic effects and side effects of the agents were evaluated by follow-up observation.

The experiment was performed by using fifty bulls and fifty cows of estimated age of one to six years old that were in diarrhea and in whose feces rotavirus antigens were detected by a conventional virus test. A group consisting of five bulls and five cows, which had been chosen at random, were orally administered with any of the above HuIFN-α agents prepared in Experiment 1 at a dose of 0.5 IU/kg body weight, once a day, for five days from the initial-administration (day 1st). Twelve-day follow-up observations were made from the initial-administration day and six clinical findings were recorded on dosage, evidence of symptom of diarrhea, colors of feces, vitality, appetite, fur and dehydration. As control, bovines were administered with 0.1 g of anhydrous crystalline maltose once a day. The bovines were fed with feedstuff containing no antimicrobial agent with unrestricted intake of water.

The experiment was chiefly conducted by a veterinarian, and the dosage of the HuIFN agent was determined with respect to condition, symptom, and side effect based on each body weight. According to the observation at twelfth day from the initial-administration day, the efficacies of the therapeutic agents were evaluated. Six general clinical findings described above were converted to scores in accordance with Table 1 to give the total clinical score of each bovine, and the average total clinical score of each group was calculated. The value obtained by subtraction of the average total clinical score of the twelfth day from the average total clinical score of the initial-administration day was divided by the average total clinical score of the initial-administration day and then multiplied by 100 to give the improvement rate (%). The results of the experiment were evaluated in four grades as the improvement rate of 85-100%, "marked efficient"; the improvement rate of 70-85%, "efficient"; the improvement rate of 50-70%, "slightly effective"; and the improvement rate of 50% or less or no improvement resulting in necessity of concomitant use of other therapeutic agents for predictable symptom exacerbations, "not effective". When categorized into "marked efficient" or "efficient", the agent was evaluated as therapeutically efficacious. In each group of HuIFN agent administration, the number of bovines evaluated as "marked efficient" or "efficient" was divided by the total number of bovines in the test group and multiplied by 100 to give the efficacy ratio (%). Among the total clinical scores, the scores of feces were separately calculated to give the average score for the feces conditions of each group, with which the improvement of feces conditions were evaluated. The total clinical scores of the experiment were calculated in accordance with Good Clinical Research Practice of antimicrobial agent for Escherichia coli diarrhea of bovine at Application for manufacturing approval *of animal drug* to Agricultural, Forestry and Fisheries Ministry of Japan. The results were shown in Table 2.

**Table 1**

| Items | Scores | | | |
|---|---|---|---|---|
| | 0 | 1 | 2 | 3 |
| Condition of feces | Normal | Soft | Muddy | Watery * |
| Color of feces | Normal | Grayish white | Milky white | |
| Vitality | Normal | Reduced | Vanished | |
| Appetite | Normal | Slightly poor | Poor | Abolished |
| Fur | Normal | Dull | Piloerect | |
| Dehydration ** | None | Mild | Moderate | Severe |

| | | | | |
|---|---|---|---|---|
| *: Including mucous and bloody feces **: Evaluated by the pinching test for the skin of the neck and the degree of the retraction of the eyes | | | | |

**Table 2**

| Kind of IFN | | Number of bovines | | | | | Efficacy ratio (%) |
|---|---|---|---|---|---|---|---|
| | | Marked effective | Effective | Slightly | Not effective | Therapeutically efficacious | |
| BALL-1 IFN | | 7 | 1 | 1 | 1 | 8 | 80 |
| Leukocyte IFN | | 3 | 1 | 4 | 2 | 4 | 40 |
| | 10:1 | 4 | 1 | 2 | 3 | 5 | 50 |
| | 9:1 | 4 | 1 | 4 | 1 | 5 | 50 |
| Mass ratio of subtype α8 and subtype α2 | 8:2 | 5 | 1 | 3 | 1 | 6 | 60 |
| | 4:6 | 5 | 2 | 2 | 1 | 7 | 70 |
| | 2:8 | 6 | 1 | 2 | 1 | 7 | 70 |
| | 1:9 | 6 | 0 | 2 | 2 | 6 | 60 |
| | 0:10 | 1 | 3 | 4 | 2 | 4 | 40 |
| Control | | 1 | 1 | 1 | 7 | 2 | 20 |

As evident from the results in Table 2, when any of the HuIFNs were administered, the clinical scores were significantly improved compared to the control, and improvement of the clinical symptoms such as diarrhea involved by the digestive system disease of bovine infected with rotavirus was observed by the oral administration of HuIFNs. When BALL-I interferon (comprising about 25% of subtype α8 and about 75% of subtype α2 to the total amount of HuIFN-α) was administered, the therapeutic effect was most significant with an efficacy ratio of 80%. When leukocyte interferon or recombinant subtype α2 alone was administered, the efficacy ratio was low as 40%, while when BALL-1 interferon or HuIFN agents comprising recombinant subtype α8 in an amount of 10% or more of the total amount of HuIFN-α (subtype αθ : subtype α2 was 1 : 9 to 10; 0) were administered, higher efficacy ratios were obtained than when leukocyte interferon or recombinant subtype α2 alone was administered. Particularly, when BALL-1 interferon or HuIFN comprising recombinant subtype α8 in an amount of 10 to 80% of and recombinant subtype α2 in an amount of 20 to 90% of the total amount of HuIFN-α (subtype α8 : subtype α2 was 1:9 to 8:2) were administered, high efficacy ratios were obtained, and when BALL-1 interferon or HuIFN comprising recombinant subtype α8 in an amount of 20 to 40% of and recombinant subtype α2 in an amount of 60 to 80% of the total amount of HuIFN-α (subtype α8 : subtype α2 was 1:9 to 4:6) were administered, particularly high efficacy ratios were obtained. The same results on the scores of improving of the feces condition were obtained as on the efficacy ratios, though the data of the scores of the feces condition were not shown. The results demonstrated that the HuIFN agents of the present invention have a therapeutic effect on digestive system diseases of bovines infected with coronavirus as well as infected by rotavirus.

During the experiment, no side effect by the administration of HuIFN agents was observed at all, it reveals that the therapeutic agent of the present invention is markedly efficient and safely used for a therapy of bovine digestive system diseases. From the results of the blood tests of some of the bovines conducted at 30th and 60th day from the initial-administration day, no antibody was produced by HuIFN-α. These results suggest that the therapeutic agents of the present invention are safe when iteratively administered to bovines for the therapy of digestive system diseases.

### Experiment 3

### Clinical test

On the basis of the above findings, this experiment was performed on calf short after birth, which are most affected by the infection of rotavirus and on which the economical problems were thought to be the largest issue, to investigate the therapeutic effect of the agents of the present invention. One hundred and forty calves (70 males and 70 females) within the six months of life infected with rotavirus and having diarrhea were administered with HuIFN agents (200 IU/g) according to the protocol of Experiment 2, and the effects and side effects were evaluated in the same way as in Experiment 2. The BALL-1 interferon agent, which marked the highest efficacy ratio in Experiment 2 was used in this experiment, and the dosages were 0.5 IU/kg body weight and 20 IU/kg body weight. For the control, 0.1g of anhydrous crystalline maltose was orally administered once a day. Sixty calves (30 males and 30 females) were used for the test of BALL-1 interferon agents, and twenty calves (10 males and 10 females) were used for the control test. The time courses of the average total clinical scores were shown in Table 3, and the average scores of feces condition were shown in Table 4, and the evaluations of the therapeutic effects were shown in Table 5.

**Table 3**

| Dosage of BALL-1 IFN (IU/Kg body weight) | Average total clinical score | | | | | | | | | | | |
|---|---|---|---|---|---|---|---|---|---|---|---|---|
| | Day from the initial administration | | | | | | | | | | | |
| | 1 | 2 | 3 | 4 | 5 | 6 | 7 | 8 | 9 | 10 | 11 | 12 |
| 0.5 | 1.9 | 1.3 | 1.0 ** | 0.7 ** | 1.0 ** | 0.9 | 1.0 | 0.8 ** | 0.6 ** | 0.6 * | 0.5 | 0.6 ** |
| 20.0 | 1.9 | 1.4 | 1.4 | 0.9 ** | 1.1 * | 0.8 ** | 0.9 * | 0.7 ** | 0.9 ** | 1.0 | 0.7 | 0.8 * |
| Control | 2.2 | 2.0 | 2.3 | 2.4 | 2.1 | 1.9 | 1.6 | 1.7 | 1.7 | 1.5 | 1.2 | 1.7 |

| | | | | | | | | | | | | |
|---|---|---|---|---|---|---|---|---|---|---|---|---|
| *: Significantly different from control (P<0.05) **: Significantly different from control (P<0.01) | | | | | | | | | | | | |

**Table 4**

| Dosage of BAIL-1 IFN (IU/Kg body weight) | Average total clinical score | | | | | | | | | | | |
|---|---|---|---|---|---|---|---|---|---|---|---|---|
| | Day from the initial administration | | | | | | | | | | | |
| | 1 | 2 | 3 | 4 | 5 | 6 | 7 | 8 | 9 | 10 | 11 | 12 |
| 0.5 | 1.4 | 1.0 * | 0.8 ** | 0.6 ** | 0.8 * | 0.8 ** | 0.9 | 0.7 ** | 0.6 ** | 0.6 ** | 0.5 | 0.6 ** |
| 20.0 | 1.5 | 1.0 * | 1.0 * | 0.7 ** | 0.9 * | 0.7 ** | 0.8 ** | 0.6 ** | 0.7 ** | 0.8 | 0.6 | 0.7 * |
| Control | 1.8 | 1.6 | 1.8 | 1.9 | 1.6 | 1.6 | 1.4 | 1.5 | 1.6 | 1.4 | 1.0 | 1.3 |

| | | | | | | | | | | | | |
|---|---|---|---|---|---|---|---|---|---|---|---|---|
| *: Significantly different from control (P<0.05) **: Significantly different from control (P<0.01) | | | | | | | | | | | | |

**Table 5**

| Dosage of BALL-1 IFN (IU/Kg body weight) | Evaluation of effect | | | | | Efficacy ratio (%) |
|---|---|---|---|---|---|---|
| | Number of calf | | | | | |
| | Marked effective | Effective | Slightly effective | Not effective | Therapeutically efficacious | |
| 0.5 | 38 | 0 | 5 | 17 | 38 | 63* |
| 20.0 | 34 | 0 | 11 | 15 . | 34 | 57* |
| Control | 5 | 1 | 2 | 12 | 6 | 30 |

| | | | | | | |
|---|---|---|---|---|---|---|
| *: Significantly different from control (P<0.01) | | | | | | |

As evident from the results in Table 3, improvement of the average total clinical score was not observed on the control group, though it fluctuated during the test. On the other hand, significant improvement was observed on the group administered with BALL-1 interferon at a dose of 0.5 IU/kg body weight in comparison with the control, from 3rd day after the initial-administration day, and the score did not become inferior at 12th day from the initial-administration day. Significant improvement was observed on the group administered with BALL-1 interferon at a dose of 20 IU/kg body weight in comparison with the control from 4th day after the initial-administration day, and the score did not become inferior at 12th day from the day of the initial administration.

As evident from the results in Table 4, the control group did not improved, though the average score of feces condition fluctuated during the test. On the other hand, significant improvement was observed on both group administered with BALL-1 interferon at a dose of 0.5 or 20 IU/kg body weight in comparison with the control from 2nd day after the day of the initial administration, and the score did not become inferior at 12th day after the day of the initial administration.

As evident from the results in Table 5, the efficacy ratio was 63% at the group administered with BALL-1 interferon at 0.5 IU/kg body weight and 57% at the group administered with BALL-1 interferon at 20 IU/kg body weight. No side effect was observed during the administration period, it indicated that the therapeutic agent of the present invention is markedly efficient and safe for the therapy of the bovine digestive system diseases. The blood test was conducted on some of the patients at 30th and 60th day from the initial-administration by the same way as Experiment 2, and no antibody production probably caused by BALL-1 interferon was detected.

These experiments demonstrated that the oral administration of HuIFN agents of the present invention are efficient antidiarrhea agent that reveals the symptom of diarrhea on the 2nd day after the initial administration and is efficient therapeutic agents for the digestive system diseases of bovines infected rotavirus or coronavirus because of no exacerbation of the clinical symptoms.

### Experiment 4

### Acute toxicity test

Bovine serum albumin was dissolved in saline containing 0.01 M of phosphate buffer (pH 7.0) to give a solution with a concentration of 0.1%. Any one of the partially purified HuIFN-α or purified HuIFN-α from BALL-1 cells prepared in Example 1, partially purified HuIFN-α or purified HUIFN-α from human leukocyte prepared in Experiment 1, and purified subtype α2 or purified subtype α8 prepared in Example 2 described below was dissolved in the above solution at a concentration of 1×10⁶ IU/ml. Seven groups of five healthy bovines, estimated age of two or three years old, were orally or subcutaneously administered with the above solutions at a dose of 1×10⁶ IU of HuIFN per kg body weight, which corresponds to 20,000-fold amount of the maximum dose of the present invention, and the follow-up observation was made. The control group was administered with the same administration route and the same amount of saline prepared by the same way as described above except that no HuIFN was dissolved.

No clinical symptom determined as side effect was observed on any group administered with any of HuIFN, the saline containing phosphate buffer dissolved with bovine serum albumin to give a concentration of 0.1 in saline. Even after the administrations, these bovines did not give any change in general conditions, body weights, or intakes and did not die. The results in the acute toxicity test, the above Experiments 2 and 3 demonstrate that HuIFN is extremely low in toxicity when administered to bovine.

The present invention is explained by the following examples. The technical scope of the present invention is not restricted by these examples.

### Example 1

In accordance with the method in Japanese Patent Kokoku No. 54158/1981, HuIPN-α was induced by subjecting BALL-1 cells, which had been grown in hamster's body, to the action of Sendai virus in a medium. The supernatant obtained by centrifuging was concentrated, and subjected to affinity-chromatography using phenyl-sepharose to give a partially purified HuIFN-α with a specific activity of 10⁶ IU/mg protein. Then the partially purified HuIFN-α was further purified by affinity-chromatography using "NK-2 sepharose (produced by Celltech Ltd.), in which a monoclonal HuIFN-α antibody was bound to a water-insoluble carrier by conventional method, and subjected to gel-filtration chromatography equilibrated with phosphate buffer (pH 7.0) containing 0.1mg/ml of bovine serum albumin to give a BALL-1 interferon solution containing a purified HuIFN-α with a specific activity of 2×10⁸ IU/mg. The HuIFN contained about 25% of subtype α8 and about 75% of subtype α2 to the total amount of HuIFN-α.

The purified HuIFN-α from BALL-1 cells prepared by the above method was concentrated to give about 5 mg protein/ml by membrane and diluted with saline containing 1% (w/v) of bovine serum albumin and 0.01 M of phosphate buffer (pH 7.0) to give a HuIFN-α concentration of 150 IU/ml. Then it was sterilized with membrane filter and aseptically dispensed in 1 ml aliquot each into vials and lyophilized.

Since this preparation contains a purified HuIFN-α form BALL-1 cells as an effective ingredient and serum albumin frombovine as vehicle, it is usable for injectable therapeutic agent for bovine digestive system diseases.

### Example 2

In accordance with the method of experiments 1-1 (a) and 1-1 (b) in Japanese Patent Kokai No. 201141/2002, a purified preparation of recombinant subtype α8 (specific activity of 3×10⁸ IU/mg protein) and a purified preparation of recombinant subtype α2 (specific activity of 8x10⁷ IU/mg protein) were prepared.

Each of the preparation of subtypes α8 and subtypes α2 prepared by the above method was concentrated to give 5 mg protein/ml. One part by weight of subtype α8 and four parts by weight of subtype α2 were mixed and the mixture was diluted to give a HuIFN-α concentration of 200 IU/ml with saline containing 1% (w/v) of officinal purified gelatin and 0.01 M of phosphate buffer (pH 7.0), then it was sterilized with membrane filter and aseptically dispensed in 1 ml aliquot each into vials and lyophilized.

This preparation contained purified recombinant subtype α8 and subtype α2 is usable as an injectable therapeutic agent for bovine digestive system diseases though it is less effective than the injectable agent of Example 1 in respect to the therapeutic effects and side effects.

### Example 3

BALL-1 interferon induced by allowing Sendai virus to act on BALL-1 cells by the same way as in Example 1 was diluted to give a HuIFN-α concentration of 2000 IU/ml with saline containing 3% (w/v) of bovine serum albumin and 0.1 M of phosphate buffer (pH 7.0), then it was sterilized with membrane filter and aseptically dispensed in 1 ml aliquot each into vials and lyophilized.

Since this preparation contains purified HuIFN-α form BALL-1 cells as an effective ingredient and serum albumin from bovine as vehicle like as the injecable agent of Example 1, it is usable as an injectable therapeutic agent for bovine digestive system diseases.

### Example 4

Bovine serum albumin was dissolved in distilled water to give a concentration of 1% (w/v), and the solution was added with the partially purified HuIFN-α induced from BALL-1 cells by the method described in Example 1 to give a HuIFN-α solution containing HuIFN-α at 300 IU/g. Thirty two parts by weight of the solution was admixed with 0.5 parts by weight of "Tween 80" dissolved in 15.0 parts by weight of ethanol and admixed with 0.5 parts by weight of triethanolamine dissolved in 15.0 parts by weight of glycerol, then added with small amount of triethanolamine to give a gelled agent containing HuIFN-α at 100 IU/ml.

This preparation containing HuIFN-α form BALL-1 calls as an effective ingredient and having an appropriate retention time in the oral cavity is a gelled oral therapeutic agent for digestive system diseases which can be easy administered to the patient.

### Example 5

Two parts by weight of the purified HuIFN-α induced from BALL-1 cells by the way described in Example 1 (about 10⁸ IU/ml) was sprayed onto one hundred parts by weight of "FTNETOSE^{®}" (anhydrous crystalline maltose commercialized by Hayashibara Shoji, Inc.), and the preparation was dried in vacuo, grinded, and sifted to give a powdery preparation in granular size of 100 to 500 pm. The powdery preparation was homogeneously admixed with adequate amount of "FINETOSE®" to give a powdery agent containing HuIFN-α at about 200 IU/g.

This preparation exhibiting an appropriate intraoral-retentivity and preservation stability can be used as a therapeutic agent for bovine digestive system diseases. This preparation is easy to be administered because of appropriate sweetness and mild flavor for administration in oral cavity of bovines.

### Example 6

A hundred parts by weight of "FINETOSE^{®}" (anhydrous crystalline maltose commercialized by Hayashibara Shoji, Inc.) was homogeneously admixed with 15 parts by weight of the solution containing the partially purified HuIFN induced from BALL-1 cells by the way described in Example 1 (about 1000 IU/ml) and the mixture was prepared into a tablet form with the tableting machine.

This preparation containing about 125 IU of HuIFN-α per 1g and exhibiting high preservation stability can be used as a therapeutic agent for bovine digestive system diseases. This preparation is easy to be administered because of appropriate sweetness and mild flavor for oral cavity of bovines.

### Example 7

Two parts by weight of the solution prepared by dissolving a "IFNβ MOCHTDA, six millions IU" (an injectable HuIFN-β commercialized by Mochida Pharmaceutical Co., Ltd.) in 1 ml of distilled water was sprayed on one hundred parts by weight of "FINETOSE^{®}" (anhydrous crystalline maltose commercialized by Hayashibara Shoji, Inc.), and the preparation was dried in vacuo, grinded, and sifted to give a powdery preparation in granular size of 100 to 500 µm. The powdery preparation was homogeneously admixed with an adequate amount of "FINETOSE^{®}" to give a powdery agent containing HuIFN-α at about 50 IU/g.

Thirty bovines of estimated age of one to six years old infected with rotavirus were administered with this preparation at a dose of 20 IU/kg body weight for 14 days in accordance with the method in Experiment 3- As an efficacy ratio of 43% was obtained, which is significantly higher than no-administration group (20 bovines were used, efficacy ratio of 30%), this preparation was evaluated as therapeutically efficacious for therapy of digestive system diseases including antidiarrhea.

Thirty bovines of estimated age of one to six years old infected with rotavirus were administered with this preparation at a dose of 10 IU/kg body weight and the agent of subtype α8 at a dose of 10 IU/kg body weight for 10 days in accordance with the method in Experiment 3. As a efficacy ratio of 52% was obtained, which is significantly higher than the no-administration group (20 bovines were used, efficacy ratio of 30%) and the group administered with the agent of subtype α8 at a dose of 20 IU/kg body weight (efficacy ratio of 45%), this preparation was evaluated as therapeutically efficacious for therapy of digestive system diseases including antidiarrhea.

This preparation exhibits an appropriate intraoral-retentivity and preservation stability. Above experimental results reveal that this preparation can be used as a therapeutic agent for bovine digestive system diseases and that the therapeutic effect is enhanced when used along with a HuIFN-α agent. This preparation is easy to be administered because of appropriate sweetness and mild flavor for oral cavity of bovines.

### INDUSTRIAL APPLICABILITY

Since HuIFN contained in the agent of the present invention is so efficient in animals when used at a low dose, the economic burden of guardians can be significantly decreased by using the agent in a small amount. For the same reason, the agent of the present invention is not necessary be administered into vessels of the animals and gives desired effects by oral or parenteral administrations such as intracutaneous, subcutaneous, or intramuscular administration. This leads to reliable and easy administration of a desired amount of HuIFN to bovines that is nervous and wary, resulting in decrease of the labor of persons in charge of the therapy including animal doctors. The agent of the present invention is so efficient for calf as to decrease economic loss caused by diarrhea of calf. The present invention, having these outstanding effects, is a significant invention that greatly contributes to the field of animal industry.

## Claims

1. A therapeutic agent for bovine digestive system diseases, comprising interferon as an effective ingredient.

2. The agent of claim 1, wherein said interferon is a human interferon.

3. The agent of claim 1, wherein said interferon is a human interferon α and human interferon β.

4. The agent of claims 2 or 3, in which the content of subtype α8 of human interferon is more than 10% (w/w) of the total amount of the interferon α.

5. The agent of any one of claims 2 to 4, in which the content of subtype α2 of human interferon is 80% (w/w) or less of the total amount of the interferon α.

6. The agent of any one of claims 1 to 5, which contains 0.005 to 5,000 international units (IU) of interferon per one g of the agent.

7. The agent of any one of claims 1 to 6, which the dosage form is selected from the group consisting of oral, transnasal, or parenteral administration.

8. The agent of any one of claims 1 to 7, wherein said digestive system disease is diarrhea caused by virus.

9. The agent of claim 8, wherein said virus is rotavirus or coronavirus.

10. A method of therapy for digestive system diseases of bovine infected with virus, which comprises a step of administering interferon to bovine at a dose of about 0.005 to 5,000 international units (IU) per one kg of body weight of the bovine.

11. The method of claim 10, wherein said interferon is a human interferon α.

12. The method of claim 10, wherein said interferon is a human interferon α and human interferon β.

13. The method of claim 11 or 12, wherein the content of subtype α8 in said interferon a is more than 10% (w/w) of the total amount of interferon α.

14. The method of any one of claims 11 to 13, wherein the content of subtype α2 in said interferon of is 80% (w/w) or less of the total amount of interferon α.

15. The method of any one of claims 11 to 14, wherein the dosage form is selected from the group consisting of oral, transnasal, or parenteral administration.

16. The method of any one of claims 11 to 15, wherein said bovine digestive system disease is diarrhea caused by virus.

17. The method of claim 16, wherein said virus is rotavirus and/or coronavirus.
